# NEW EUROPEAN PATENT SPECIFICATION

(11) **EP 3 411 057 B2**
(45) Date of publication and mention of the opposition decision: **20.12.2023**
(45) Mention of the grant of the patent: 16.12.2020
(21) Application number: 17702403.1
(22) Date of filing: 03.02.2017
(51) Int. Cl.: A23J 3/34, A23J 1/20, A23L 33/17, A23L 33/00, A61K 38/01, A61K 47/42

(54) **HIGH CALORIC, HIGH PROTEIN NUTRITIONAL FORMULA COMPRISING COLLAGEN**
KALORIEN- UND PROTEINREICHE ERNÄHRUNGSFORMEL UMFASSEND KOLLAGEN
FORMULE NUTRITIONELLE, HYPERCALORIC ET HYPERPROTIDIQUE COMPRENANT DE LA COLLAGÈNE

(30) Priority: 03.02.2016 EP 16154090; 18.08.2016 EP 16184825
(43) Date of publication of application: 12.12.2018
(60) Divisional application: 20211750.3
(73) Proprietor: Fresenius Kabi Deutschland GmbH, 61352 Bad Homburg (DE)
(72) Inventor: BRITO DE LA FUENTE, Edmundo, 61381 Friedrichsdorf (DE); KEIM, Susanne, 61350 Bad Homburg (DE); PESTANA, Ericka, 61352 Bad Homburg (DE); MAINOU-SIERRA, José Maria, 61239 Ober-Moerlen (DE); MARTINEZ-BOCK, Maria Fernanda, 60327 Frankfurt am Main (DE); WALLENFELS, Eileen, 35466 Rabenau (DE); WIEGAND, Susanne, 60599 Frankfurt am Main (DE)
(74) Representative: Fresenius Kabi Deutschland GmbH
(86) International application number: PCT/EP2017/052428
(87) International publication number: WO 2017/134245

(56) References cited:
- WO-A1-95/22909
- WO-A1-2005/023017
- WO-A1-2005/092120
- WO-A1-2015/095542
- WO-A1-2016/177775
- CA-A- 967 053
- CZ-U1- 27 640
- DE-A1-102010 060 564
- JP-A- 2001 061 444
- JP-A- 2008 247 748
- US-B1- 6 383 534
- ANONYMOUS: "Milupa Pregomin / AS", INTERNET CITATION, 30 March 2004 (2004-03-30), XP002308177, Retrieved from the Internet: URL:http://www.milupa.de/wms/pdf/ik_pregom in_20040330.pdf [retrieved on 2004-11-30]
- Fabiane La ET AL: "MISTURAS DE PROTEÍNAS DE SORO LÁCTEO E COLÁGENO | 61", Revista de Nutrição, 1 January 2009 (2009-01-01), pages 61-70, XP055259276, Retrieved from the Internet: URL:http://www.scielo.br/pdf/rn/v22n1/06.p df
- GMEZ-GUILLN M C ET AL: "Functional and bioactive properties of collagen and gelatin from alternative sources: A review", FOOD HYDROCOLLOIDS, ELSEVIER BV, NL, vol. 25, no. 8, 8 February 2011 (2011-02-08), pages 1813-1827, XP028255533, ISSN: 0268-005X, DOI: 10.1016/J.FOODHYD.2011.02.007 [retrieved on 2011-02-15]
- Patel Kamlesh C et al: "The Temperature Uniformity During Air Drying of a Colloidal Liquid Droplet", Drying Technology, vol. 23, 2005, pages 2337-2367,
- Dairy Export Council U S: "Nutritional composition of skim milk powder", Reference Manual for U.S. Milk powders, 2005, page 41,
- Wikipedia: Molar mass distributionhttps://en.wikipedia.org/wiki/ Molar mass distribution https://en.wikipedia.org/wiki/Molar_mass_d istribution
- Polymerdatabase.com: Molecular weight and molecular weight distribution https://polymerdatabase.com/polymer%20phys ics/Molecular%20Weight.html
- Dried dairy ingredients, Karen Smith, Wisconsin Center for Dairy Research, May 15, 2008
- The Food Energy Value of Polyols, European Association of Polyol Producers https://polyols-eu.org/wpcontent/uploads/F ood_energy_value_of_polyols.pdf#:~:text=Th e%20Food%20Energy%20Value%20of%20Polyols%2 0The%20Food,The%20energy%20value% 20for%20sugar%20is%2017kJ%2Fgram%20%284kca l%2Fgram%29

## Description

### FIELD OF THE INVENTION

The present disclosure relates to ready-to-use nutritional compositions of high caloric density and high protein content. The compositions herein further comprise a lipid component, a carbohydrate component, vitamins, minerals, water and, preferably, are nutritionally complete. The present disclosure further relates to a process for producing such a composition. Such nutritional compositions are suitable to provide homogeneous nutritionally complete emulsions even at very high energy densities (e.g. at least 2.5 kcal/mL, preferably at least 3 kcal/mL) in combination with very high protein densities (at least 14wt%) based on the total weight of the composition).

### BACKGROUND OF THE INVENTION

There is a general need for nutritional compositions providing a high amount of calories and nutrients in a relatively low volume of formula.

Such nutritional compositions are particularly needed for elderly patients having reduced appetite, for patients with reduced tolerance to high or even normal amounts of food, patients in need of more than normal calorie and nutrient supply (e.g. patients suffering from (chronic) wasting diseases).

One issue in feeding patients at risk of or with malnutrition is low compliance with the nutritional therapy. Patients often do not tolerate high or even normal volumes of food and / or nutritional compositions.

Typically, the need for a high amount of calories in relatively low volume is met by increasing the amount of lipid and/ or carbohydrate in nutritional compositions. Increasing the protein content is difficult. In particular in compositions comprising minerals and vitamins, such as nutritionally complete compositions, interactions between minerals and proteins make it often impossible to obtain a stable, homogeneous composition.

However, there is often a medical need for compositions being high in protein and calories in a low volume of formula. And still, for mainly technical reasons, nutritional compositions having a high amount of protein typically have a lower caloric density. Also, compositions high in protein are often devoid of or at least low in lipids and/ or carbohydrates.

Thus, the protein density of a nutritional composition is typically not simultaneously increased with the caloric density as such an increase typically poses problems in terms of processability, homogenization, sterilization, fouling, instable emulsions and/or high viscosity. These issues are most pronounced for nutritionally complete compositions, i.e. compositions providing minerals and vitamins in amounts rendering them suitable as sole source of nutrition.

Therefore, when it comes to ready to use nutritional compositions and in particular nutritionally complete compositions, the patient / caregiver typically has to choose one of two options: 1. high caloric nutrition or 2. nutrition high in protein.

CZ27640 U1 discloses nutritional compositions for diabetics comprising a high proportion of whey protein and hydrolysed collagen.

Accordingly, there is a need for nutritional compositions simultaneously providing a high caloric density and a high amount of protein. In particular there is a need for such a composition being additionally adaptable to be nutritionally complete.

### SUMMARY OF THE INVENTION

The inventors found that the use of a protein component comprising at least two different protein sources, wherein collagen hydrolysate with an average molecular weight M_{w} of from 1000 Da to 6000 Da is the first protein source and represents 70-90 wt% based on the total weight of the protein component, and wherein the second protein source is selected from milk proteins and represents 10-30 wt% based on the total weight of the protein component, and wherein the nutritional composition has a caloric density of at least 2 kcal/mL, and wherein the nutritional composition comprises at least 14g protein per 100mL product, it is possible to provide homogeneous nutritional compositions having both: high caloric density and a high amount of protein in a low volume.

Moreover, the inventors found that such nutritional compositions can be supplemented with minerals and vitamins in sufficient amounts to render them nutritionally complete and still be stable and homogeneous.

In a first aspect, the present disclosure relates to nutritional compositions comprising a protein component, a lipid component, a carbohydrate component, minerals, vitamins and water, wherein the protein component comprises at least two different protein sources, wherein the nutritional composition has a pH in the range of 5.5 - 9.0, wherein the nutritional composition has a caloric density of at least 2 kcal/mL, wherein the nutritional composition comprises at least 14g protein per 100ml product, and wherein the first protein source is hydrolysed collagen with an average molecular weight M_{w} of from 1000 Da to 6000 Da, the lipid component provides at least 30 EN % of the total energy of the composition and the first source of protein represents 70-90 wt% based on the total weight of the protein component, and wherein the second protein source is milk protein and present at 10-30 wt% based on the total weight of the protein component.

In a second aspect, the present disclosure relates to a process for making such a nutritional composition.

### DETAILED DESCRIPTION OF THE INVENTION

### Definitions

"High protein" as used herein refers to nutritional compositions wherein the protein component provides at least 15 EN%, preferably at least 18 EN%, most preferred at least 20 EN% based on the total energy of the nutritional composition. In preferred embodiments, such nutritional compositions comprise at least 10 wt%, preferably at least 12wt%, more preferably at least 14 wt% of protein based on the total weight of the composition. According to the present disclosure, there may be an upper limit to protein content such as at most 40 EN%, preferably at most 30 EN%, more preferred at most 28EN%.

"High caloric" as used herein refers to nutritional compositions having a caloric density of at least 2 kcal/mL, preferably at least 2.5 kcal/ml, more preferably at least 2.8 kcal/mL, most preferably at least 3.0 kcal/mL.

"Hydrolysed collagen" or "collagen hydrolysate" as used herein refers to low molecular weight collagen obtainable by hydrolysis of collagen by procedures known to the skilled artisan. Hydrolysed collagen as used herein has an average molecular weight M_{w} of from 1000 Da to 6000 Da, preferably 1000 to 3000 Da. Methods for determining the average molecular weight are well known in the art. An exemplary method is given hereinbelow.

"Nutritional composition" herein refers to a synthetically produced food composition. Thus, nutritional compositions are artificial nutritional products obtained by mixing / dissolving bulk ingredients whereby said ingredients are typically provided in solid form (e.g. powders) or liquid from (e.g. oils, water, syrup). The term "nutritional composition" excludes "food", i.e. non-modified natural food products, such as meat, vegetables, fruits in their natural form and conventionally prepared (e.g. cooked) meals or drinks like tea, coffee or juices.

For the present disclosure, "nutritional compositions" are limited to liquid or semi-solid compositions.

"Patient nutrition" as used herein refers to nutrition intended for individuals suffering from a medical condition. Patient nutrition as defined herein excludes the provision of nutrients in the form of conventionally prepared meals ("food"). Patient nutrition therefore only refers to the provision of nutrients in form of nutritional compositions as defined above. Herein, patient nutrition is intended for patients having high caloric needs and high protein needs.

"Nutritionally complete" refers to nutritional compositions suitable as sole source of nutrition. To be nutritionally complete, it is required that a nutritional composition comprises - in addition to the lipid, carbohydrate and protein components - minerals and vitamins. In order to be nutritionally complete, vitamins and minerals should be present in sufficient amounts as known to the man skilled in the art, i.e. in accordance with established nutritional guidelines. The recommended nutrient requirements, in particular with respect to minerals and vitamins, can be found in standard nutritional guidelines such as EU commission directive 1999/21/EC (see table 1 hereinbelow). Suitable nutrients according to the present disclosure fulfil the requirements of / are listed in REGULATION (EU) No 609/2013.

"Malnutrition" as used herein refers to one or both of Option I: body mass index (BMI, kg/m2) <18.5; Option II: the combined finding of unintentional weight loss (mandatory) and at least one of either reduced BMI or a low fat free mass index (FFMI). Weight loss is defined as either >10% of habitual weight indefinite of time, or >5% over 3 months. Reduced BMI is <20 or <22 kg/m2 in subjects younger and older than 70 years, respectively. Low FFMI is <15 and <17 kg/m2 in females and males, respectively.

A composition "consisting of" a number of ingredients or components is to be understood as comprising no other than the named ingredients or components. In case ranges for amounts of ingredients or components are given, the individual amount of all ingredients or components within the composition has of course also to be adapted such that the sum of all amounts of all present ingredients or components adds up to 100wt%.

"Homogenization" as used herein refers to the process of diminishing the size of the fat globules in a nutritional emulsion. A preferred homogenization process herein comprises two homogenization steps. The first homogenization step may be carried out at a pressure of 100bar and the second homogenization step may be carried out at 50 bar. Both steps may be carried out at a temperature of 60-80°C, such as 65-75°C, for example 65 °C.

"UHT-treatment" aims at killing of microorganisms. Preferred UHT treatment may be carried out with a pre-heat treatment at 90°C for 3 min followed by UHT at 139°C for 6 seconds, followed by a (third) homogenization step requiring homogenization at less than 90°C with a pressure that can oscillate between 40-150 bar.

"Protein component" as used herein refers to the entirety of ingredients of the nutritional composition declarable as "protein".

"Lipid component" as used herein refers to the entirety of ingredients of the nutritional composition declarable as "fat".

"Carbohydrate component" as used herein refers to the entirety of ingredients of the nutritional composition declarable as "carbohydrate".

"EN%" refers to the contribution of a certain component or of a specific ingredient to the total nutritional energy of an edible composition, e. g the nutritional composition.

"Ready-to-use" refers to the final form of the nutritional composition as administered to a patient. Typically, the nutritional compositions herein are pre-packed in a ready to use format. I.e. sold in separately packed dose units that do not require any further dilution etc..

### Nutritional compositions

The nutritional compositions herein comprise nutrients in predetermined and controllable amounts. A nutritional composition according to the present disclosure comprises a protein component, a lipid component, a carbohydrate component, minerals, vitamins and water. Optionally, such a nutritional composition may further comprise dietary fibres and/or further ingredients known as food additives.

The nutritional compositions herein typically are liquid or semi-solid and will typically be provided in an oil-in-water emulsion (O/W).

The nutritional compositions are adapted to have a high caloric density of at least 2 kcal/mL, preferably at least 2.5 kcal/ml, more preferably at least 2.8 kcal/mL, most preferably at least 3.0 kcal/mL. Typically, the nutritional compositions will have a caloric density of at most 5.0 kcal/mL, preferably at most 4 kcal/ml, most preferred at most 3.8 kcal/mL. For example, the compositions herein have a caloric density of 3.0-4.0 kcal/mL.

The nutritional composition herein may comprise a lipid component, a protein component, a carbohydrate component, wherein
a. the protein component provides at least 15 EN %, preferably at least 18 EN%, more preferably at least 20 EN% of the total energy of the composition
b. the lipid component provides at least 30 EN % of the total energy of the composition;
c. the carbohydrate component provides at least 20 EN % of the total energy of the composition;

Preferably, in the nutritional compositions according to the present disclosure comprises a lipid component, a protein component, a carbohydrate component, wherein
a. the protein component provides 15-25 EN%, preferably 18-22 EN%;
b. the lipid component provides 40-50 EN%, preferably 43 - 47 EN%; and
c. the carbohydrate component provides 30-40 EN% preferably 33-37 EN%.

In preferred embodiments, the amount of water comprised in the present composition represents 40-60 wt% based on the total weight of the nutritional composition, preferably 45-55 wt%.

The nutritional composition of the present disclosure is administered enterally, preferably orally.

### Protein component

The protein component comprises at least two different protein sources, wherein the first protein source is hydrolysed collagen with an average molecular weight M_{w} of from 1000 Da to 6000 Da and represents 70-90 wt%, more preferably 80-85 wt% based on the total weight of the protein component.

Comparing nutritional compositions comprising high amounts of hydrolysed protein the present nutritional compositions (comprising hydrolysed collagen with an average molecular weight M_{w} of from 1000 Da to 6000 Da) should lead to improved patient compliance due to improved rheological and sensorial properties like viscosity, texture and / or taste.

The second protein source is milk protein, wherein the milk protein represents 10-30 wt% based on the total weight of the protein component

Within the second protein source, proteins having different average molecular weights may be used. Preferred average molecular weights (M_{w}) of the proteins used within the second protein source lie in the range of 20-60kDa. In such a range properties of the nutritional composition can be well balanced in terms of heat stability and/ or viscosity.

For example, a second protein source comprising a high amount of a protein having a lower molecular weight will lead to a reduced viscosity of the nutritional composition. Therefore, a preferred second protein source comprises more than 40 wt%, preferably more than 50 wt% of a protein having an average molecular weight of less than 40kDa, such as 20-40 kDa (based on the total weight of the second protein source).

The protein component comprises collagen hydrolysate with an average molecular weight M_{w} of from 1000 Da to 6000 Da as the first protein source, wherein the first source of protein represents 70-90 wt% based on the total weight of the protein component and milk protein as the second protein source, wherein the milk protein represents 10-30 wt% based on the total weight of the protein component. Such a protein component is particularly suitable as it can be adapted such that it provides an amino acid distribution suitable to meet current international recommendations for daily intake (e.g. when the nutritional compositions of the present disclosure are used as sole source of nutrition), even without addition of free amino acids, di- or tri-peptides. Such an exemplary international recommendation has been published by the WHO (Technical Report Series 935, 2007, p. 150). Particularly preferred is a protein component comprising 80 wt% hydrolysed collagen with an average molecular weight M_{w} of from 1000 Da to 6000 Da with 20 wt% milk protein (each based on the total weight of the protein component).

Further amino acids may as well contribute to the second protein source. These may be added in their chemical form or in the form of low molecular peptides, such as di- or tri-peptides. However, in preferred embodiments neither free amino acids nor di- or tri peptides are added to the protein component described herein.

Nutritional compositions herein typically comprise at least 10 wt%, preferably at least 12 wt%, more preferably at least 14 wt% of protein based on the total weight of the nutritional composition.

In preferred embodiments, the protein component provides at least 15 EN%, preferably at least 18 EN%, more preferably at least 20 EN% based on the total energy of the nutritional composition. For example the protein component provides 15-25 EN%, preferably 18-22 EN% of the total energy of the composition.

Preferably, the protein to water ratio of the present nutritional composition is at least 2.0/10 [g/g], preferably at least 2.5/10 [g/g].

### Collagen hydrolysate

As described above, the collagen hydrolysate herein has an average molecular weight M_{w} of from 1000 Da to 6000 Da, preferably 1000 to 3000 Da. Examples are known to the man skilled in the art and commercially available (e.g. via Gelita, Germany). Such hydrolysates and methods for making them are for example described in DE 102010060564 A1, in particular [0004] - [0011] and Example 1 with the low molecular weight hydrolysates in par. [0030] being particularly suitable. Of course, other sources than procine gelating can be used, with bovine being particularly preferred for the applications herein.

Accordingly, particularly preferred collagen hydrolysates have molecular weight distributions listed below:

| Mol. weight range | Upper limit |
|---|---|
| > 7500 Da | 10 wt% |
| > 3500 - 7500 Da | 35 wt% |
| > 1500 - 3500 Da | 35 wt% |
| > 500 - 1500 Da | 50 wt% |
| < 500 Da | 15 wt% |

| Mol. weight range | Preferred range | Particularly preferred range | Example 1 | Example 2 |
|---|---|---|---|---|
| > 7500 Da | ≤10 wt% | ≤5 wt% | ≤5 wt% | ≤5 wt% |
| > 3500 - 7500 Da | 10-35 wt% | 10 - 20 wt% | 10 - 20 wt% | 12 - 18 wt% |
| > 1500 - 3500 Da | 20-35 wt% | 25 - 32 wt% | 25 - 32 wt% | 25 - 31 wt% |
| > 500 - 1500 Da | 30-50 wt% | 40 - 50 wt% | 40 - 50 wt% | 40 - 46 wt% |
| < 500 Da | ≤15 wt% | ≤15 wt% | ≤15 wt% | 5 -10 wt% |

Preferably, at most 10 wt%, more preferably at most 5 wt% of the collagen hydrolysate has a molecular weight of above 7,500 Da.

Preferably, at most 15 wt%, more preferably at most 5 wt% of the collagen hydrolysate has a molecular weight of below 500 Da.

Thus, particularly preferred collagen hydrolysates are characterized by at least 75 wt%, preferably at least 90 wt% falling into the molecular weight range of 500-7500 Da.

### Carbohydrate component

The carbohydrate component may comprise one or more carbohydrate sources. Typical carbohydrate sources may be selected from the list consisting of maltodextrine, glucose syrup, sucrose, fructose, isomaltulose, starch (modified or unmodified), tapioca dextrine, and mixtures thereof.

Typically, in nutritional compositions herein the carbohydrate component provides at least 25 EN%, preferably 30-40 EN%, for example 33-37 EN% based on the total energy of the nutritional composition.

A preferred carbohydrate component comprises glucose syrup and, preferably sucrose. The carbohydrate component may comprise 65-95wt% of glucose syrup and 35-95wt% of sucrose based on the total weight of the carbohydrate component. Preferably, the carbohydrate component comprises 60-80wt% glucose syrup and 20-40wt% sucrose, for example 65-75wt% glucose syrup and 25-35wt% sucrose based on the total weight of the carbohydrate component.

### Fibre

The nutritional composition herein may comprise ingredients declarable as dietary fibres. Suitable dietary fibres may be selected from the group consisting of cocoa powder, inulin, wheat dextrine, cellulose, microcrystalline cellulose, soy polysaccharides, tapioca dextrine, xanthan, fructooligosaccharides, galactooligosaccharides, at least partially hydrolysed guar gum, acacia gum, pectin, oat fibre, poly dextrose, resistant starch, hemicellulose and mixtures thereof.

### Lipid component

The lipid component may comprise one or more lipid sources, such as lipids of animal and / or vegetable origin. Suitable lipid sources may be selected from oil of marine origin vegetable oils and combinations thereof. Preferably, lipid sources may be selected from fish oil, sunflower oil, safflower oil, soy oil, rapeseed oil, canola oil, linseed oil and combinations thereof. Additionally, the lipid component may comprise MCT in oil or fat form providing C6-C12 fatty acids.

In terms of individual fatty acids, the lipid component typically includes polyunsaturated fatty acids, monounsaturated fatty acids and saturated fatty acids. Suitable fatty acids may be selected from the group consisting of caproic acid (C6:0), caprylic acid (C8:0), capric acid (C10:0), lauric acid (C12:0), myristic acid (C14:0), palmitic acid (C16:0), palmitoleic acid (C16:1w7), stearic acid (C18:0), oleic acid (C18:1w9), linoleic acid (C18:2w6), a-Linolenic acid (C18:3w3), eicosapentaenoic acid (C20:5w3), docosahexaenoic acid (C22:6w3) and mixtures thereof.

The lipid component provides at least 30 EN%, preferably at least 40 EN% of the total energy of the nutritional composition herein.

In preferred embodiments, the lipid component provides at most 50 EN%.

Preferably, the lipid component provides 40-50 EN%, for example 43 - 47 EN% based on the total energy of the nutritional composition.

### Vitamins and Minerals

To be regarded as nutritionally complete, nutritional compositions have to comprise vitamins and minerals.

Suitable vitamins to be included in the composition in order to render it nutritionally complete according to the present disclosure are Vitamin A, Vitamin D, Vitamin K, Vitamin C, Thiamin, Riboflavin, Vitamin B6, Niacin, Folic acid, Vitamin B12, Pantothenic acid, Biotin and Vitamin E. An example for rendering a nutritional composition complete in vitamins is given in table 2.

Suitable minerals to be included in the composition in order to render it nutritionally complete according to the present disclosure are Sodium, Chloride, Potassium, Calcium, Phosphorus, Magnesium, Iron, Zinc, Copper, Iodine, Selenium, Manganese, Chromium and Molybdenum. Optionally, Fluoride may be included. An example for rendering a nutritional composition complete in minerals is given in table 3.

### Additives

Nutritional compositions optionally comprise food additives. Additives are typically present in total amount of less than 10wt%, 5wt% or even less than 1wt% based on the total weight of the nutritional composition. Exemplary additives are choline, beta-carotene, lutein, lycopene, caffeine, lecithin, taurine, carnitine, myo-inositol, colorants, aroma and mixtures thereof. Aromas may be caramel, vanilla, yoghurt, chocolate, coffee, cappuccino, fruit aromas and the like.

The additives may include the stabilisers and emulsifiers. Preferably, the stabilisers are selected from gums and mixtures thereof. For example microcrystalline cellulose (E460), sodium carboxymethylcellulose (E466), carrageenan (E407), diacteyl tartaric acid ester of glycerides, cellulose gel (cellulose, microcrystalline). The emulsifiers may be selected from (destilled) monoglycerides such as E471, soy lecithins. For example, stabilizers and emulsifiers are included in the following amounts / ratios monoglycerides (E471, as an emulsifier) 1-5g/L and a stabilizer mixture comprising 0.3-5g/L, soy lecithin 1-5g/L, diacetly tartaric acid of glycerides (E472, eg. DATEM) 0.1-5 g/L and MCC 1-8g/L.

### Use in therapy

The nutritional composition of the present disclosure may be used in therapy.

For example, the nutritional compositions of the present disclosure may be used in nutritional therapy. In other words, the nutritional composition herein may be used as patient nutrition.

In one embodiment, the nutritional compositions of the present disclosure are for use in prevention and treatment of nutrient deficiencies associated with malnutrition.

In a further embodiment the nutritional compositions of the present disclosure are for use in prevention and treatment of nutrient deficiencies associated with therapeutic fluid restriction. For example, patients requiring therapeutic fluid restriction may be selected from dialysis patients, patients with cardiac insufficiency and patients with liver insufficiency.

In a further embodiment the nutritional compositions of the present disclosure are for use in prevention and treatment of nutrient deficiencies associated with (chronic) wasting diseases such as catabolism, cachexia, including cancer cachexia or cardiac cachexia, HIV, chronic wounds or sarcopenia. The nutritional compositions of the present disclosure are for use in prevention and treatment of nutrient deficiencies in any condition associated with increased protein needs or overall energy, preferably both. Examples are use in prevention and treatment of nutrient deficiencies in gastrointestinal conditions associated with protein and / or energy wasting. Other examples are use in prevention and treatment of nutrient deficiencies of surgical patients, use in prevention and treatment of nutrient deficiencies associated with wounds and pressure ulcers or use in prevention and treatment of nutrient deficiencies associated with cancers.

In one embodiment, the nutritional compositions of the present disclosure are for use in prevention and treatment of protein-energy deficiencies in patients suffering from conditions associated with such deficiencies.

Generally, the nutritional compositions herein are for use in prevention and treatment in protein-energy malnutrition (PEM). Thus, the nutritional compositions herein are for use in treatment patients with or at risk of protein malnutrition or PEM. Patients can be at risk of PEM in view of a general condition associated with protein wasting, or in view of inadequate dietary protein or protein and calorie intake. Patients can be considered at risk of PEM when consuming diets with inadequate protein intake for prolonged periods of time. For example, patients consuming diets with less than 10EN% of protein or patients neither meeting neither protein nor calorie requirements.

The nutritional compositions herein are particularly suitable for prevention and treatment of protein-energy deficiencies of elderly patients, pregnant or lactating patients and children (age >1 year). For example for prevention and treatment of protein-energy deficiencies of elderly patients (age > 65yrs), pregnant or lactating patients and children (age >1 year), diagnosed with or at risk of PEM.

In one embodiment, the nutritional compositions of the present disclosure are for use in prevention and treatment of nutrient deficiencies, in particular protein-energy deficiencies associated with frailty and / or sarcopenia in elderly patients (age >65), more preferably hospitalized patients (including patients in nursing homes).

Moreover, the nutritional compositions of the present disclosure are for use in prevention and treatment of nutrient deficiencies associated with neurological disorders, anorexia, chronic obstructive pulmonary disease (COPD).

### Preparation of nutritional compositions

The nutritional compositions described herein are obtainable by a process comprising the following steps as defined in independent claim 10.

In a first step, water is provided and heated to a temperature of 50-70°C.

In a second step the carbohydrate component - or a fraction thereof - and, optionally, stabiliser, such as MCC, is added.

In a third step the protein component is added.

In a fourth step the lipid component is added, preferably at an elevated temperature of 80-90°C, preferably in combination with a (further) emulsifier and / or stabiliser, such as soy lecithin and / or diacetyl tartric acid of glycerides (e.g. DATEM), and / or (destilled) monoglycerides.

In a fifth step vitamins, minerals and, optionally, the remaining fraction of the carbohydrate component are added. In this step any other powder ingredient may be added. E.g. fibres, aroma, and other additives. At any time before, while or after the fifth step, the mixture is allowed to cool to 55-65°C.

In a sixth step, the pH of the mixture is adjusted to 6-9, preferably 7-8, e.g. 7.

In a seventh step, the mixture is homogenized, e.g. at 60-65°C at a pressure of 100/50 bar,

In an eighth step, the mixture is sterilized. The eighth step may comprise (8.a) a pre-heating step, (8.b) a heating step and (8.c) an additional homogenization step. For example:
a. in step 8.a the mixture is pre-heated to 70-100°C, preferably 85-95°C, e.g. for 2-5 minutes;
b. in step 8.b the mixture is heated to 130-140°C, preferably to 139-141°C, e.g. for 6-15 seconds; and
c. in step 8c the mixture is homogenized at a pressure of 40-200bar, preferably step 8.c is carried out at less than 90°C.

In an alternative embodiment, all or a part of the additional ingredients, such as aroma, may be added already between the third and fourth step.

### Dose unit and daily dose

The nutritional compositions herein are typically provided in a dose unit.

A dose unit herein refers to 100-200mL, preferably 100-150mL separately provided in package such as a bottle, tetra brick or bag.

Such a dose unit provides 300 - 500kcal, preferably 350-450kcal.

Such a dose unit provides 14-40g, preferably 15-30g, more preferably 18-22g of protein, such as 20g of protein.

An exemplary dose unit provides 400kcal and 20g of protein in a dose unit of 125mL.

Accordingly, an exemplary daily dose for complete nutrition of 1500-2500kcal of the nutritional compositions herein may be provided with 3-8 dose units, for example 5 dose units. Accordingly, a daily dose for complete enteral nutrition may be provided by 5 dose units each providing 350-450kcal,

A typical daily dose for supplemental nutrition of 300-900kcal of the nutritional compositions herein will be provided by 1-3 dose units. For example by 1-2 dose units each providing 350-450kcal.

### EXEMPLES

Nutritional compositions according to table 1 were prepared. After mixing, a homogenization step was conducted.

Example 1 is a standard nutritional composition not according to the invention already high in calories with a protein component solely based on milk protein. While being stable and homogeneous at 2.0 kcal and 20EN% protein (Example 1), it was not possible to obtain a sufficiently homogeneous composition when increasing caloric density (Example 2). Example 2 is not according to the present invention.

Surprisingly, increasing caloric density was possible by adding collagen hydrolysate in sufficient amounts to milk protein (Example 4 and Example 5). Example 3 is a comparative example with insufficient amount of collagen hydrolysate. Example 4 is also a comparative example.

Example 4 further shows that with inclusion of sufficient collagen hydrolysate very high energy densities of protein can be reached in a formula that provides high caloric density (Example 4: 2.6 kcal) or very high caloric density (Example 5: 3.2 kcal/mL).

The inventors further observed that fouling during heat treatment could be reduced / prevented when increasing wt% of collagen hydrolysate in the protein component (Example 5).

With the nutritional composition, a drinkable viscosity of nutritionally complete compositions can be provided inspite high protein and energy content.

The inventors further observed that bitterness of the product could be reduced by increasing wt% of collagen hydrolysate in the protein component. Therefore, the nutritional composition will result in a better patient compliance.

### Trial

The nutritional composition according to Example 5 (test drink) is given to subjects with an indication for supplemental nutrition of approx. 400kcal per day. Subjects belong to the age group of 65 and older. Age, sex, height and BMI are recorded. The dosage is one bottle of 125ml per day for seven consecutive days. One such bottle provides 400kcal, including 20g protein (20EN%), 20g fat (45EN%) and 35g carbohydrates (35EN%).

Adverse effects are documented. Gastrointestinal tolerance parameters are documented. Moreover, (protein) energy intake, compliance (in particular time until full consumption of the test drink and / or amount consumed within 1 hour), activity level and palatability are documented.

### METHODS

### Determination of molecular weight of the hydrolysed collagen by GPC/HPLC

Equipment: GPV/HPLC with UV detector operating at 214 nm. Column: TSK 2000 SW XL (Toshoh Biosience GmbH). Isocratic elution using 400 mmol/l sodium phosphate buffer (pH 5.3). Calibration by means of well-defined Type I-collagen fragments (FILK, Freiburg, Germany). The collagen hydrolysate used in the examples had an average molecular weight of 2kDa. In general, the skilled person is well aware of molecular mass determination via GPC. Another suitable method for determining the M_{w} of small macromolecules such as the hydrolysates described herein is MALDI- MS.

**TABLE 1: Exempels**

| | Example 1 | Example 2 | Example 3 | Example 4 | Example 5 |
|---|---|---|---|---|---|
| Protein source 1 | -/- | - /- | Collagen hydrolysate 28.6% | Collagen hydrolysate 49% | Collagen hydrolysate 80% |
| Protein Source 2 | Milk protein 100% (Refit 54% MPC-80 46%) | Milk protein 100% (Refit^{®} MCI 88) | Milk protein 71.4% (Refit 38.8% MPC-80 32.6%) | Milk protein 51% (Refit 28% MPC-80 23%) | Milk protein 20% (Refit 10,8% / MPC-80 9.2%) |
| Protein | 20 EN% | 20 EN% | 26 EN% | 20 EN% | 20 EN% |
| CHO | 35 EN% | 30 EN% | 33 EN% | 35 EN% | 35 EN% |
| Fat | 45 EN% | 50 EN% | 41 EN% | 45 EN% | 45 EN% |
| Caloric density | 2.0 kcal | 3.2kcal / L | 3.2kcal / L | 2.6 kcal | 3.2kcal / L |
| FSMP balanced * | yes | No | No | yes | yes |
| Homogenization method (1^{st} step / 2^{nd} step) | 100 / 50 bar | 100 / 50 bar | 100 / 50 bar | 100 / 50 bar | 100 / 50 bar |
| Homogenization / heat treatment | homogeneous | Not possible to homogenize. Consequently, no heat treatment was applied. | Possible to homogenize. However the matrix was not stable during Autoclave Heat Treatment, the product became a curdly mass with expelled water. | Possible to homogenize. In case of (optional) subsequent UHT sterilization, fouling in the line was observed. | Possible to homogenize. Compared to example 4: Improved stability against subsequent UHT sterilization, no fouling. - Lower viscosity Improved shelf life (i.e. at least ? months) |
| * Nutritionally complete in vitamins and minerals | | | | | |

**TABLE 2: Vitamins**

| | Minimum per 100kcal | Maximum per 100kcal |
|---|---|---|
| Vitamin A (µg RE) | 35 | 180 |
| Vitamin D (µg) | 0,5 | 3 |
| Vitamin K (µg) | 3,5 | 20 |
| Vitamin C (mg) | 2,2 | 22 |
| Thiamin (mg) | 0,06 | 0,5 |
| Riboflavin (mg) | 0,08 | 0,5 |
| Vitamin B6 (mg) | 0,08 | 0,5 |
| Niacin (mg EN) | 0,9 | 3 |
| Folic acid (µg) | 10 | 50 |
| Vitamin B12 (µg) | 0,07 | 0,7 |
| Pantothenic acid (mg) | 0,15 | 1,5 |
| Biotin (µg) | 0,75 | 7,5 |
| Vitamin E (mg α-TE) | 0,5 | 3 |

**TABLE 3: Minerals**

| | Minimum per 100kcal | Maximum per 100kcal |
|---|---|---|
| Sodium (mg) | 30 | 175 |
| Chloride (mg) | 30 | 175 |
| Potassium (mg) | 80 | 295 |
| Calcium (mg) | 35 | 250 |
| Phosphorus (mg) | 30 | 80 |
| Magnesium (mg) | 7,5 | 25 |
| Iron (mg) | 0,5 | 2,0 |
| Zinc (mg) | 0,5 | 1,5 |
| Copper (µg) | 60 | 500 |
| Iodine (µg) | 6,5 | 35 |
| Selenium (µg) | 2,5 | 10 |
| Manganese (mg) | 0,05 | 0,5 |
| Chromium (µg) | 1,25 | 15 |
| Molybdenum (µg) | 3,5 | 18 |
| Flouride (mg) | - | 0,2 |

## Claims

1. Nutritional composition comprising a protein component, a lipid component, a carbohydrate component, minerals, vitamins and water, wherein the protein component comprises at least two different protein sources; wherein the nutritional composition has a pH in the range of 5.5 - 9.0, wherein the nutritional composition has a caloric density of at least 2 kcal/mL, wherein the nutritional composition comprises at least 14g protein per 100mL product, and wherein
a) the first protein source is hydrolysed collagen with an average molecular weight M_{w} of from 1000 Da to 6000 Da
b) the lipid component provides at least 30 EN% of the total energy of the composition
c) the first source of protein represents 70-90 wt% based on the total weight of the protein component and
d) the second protein source is milk protein and wherein the milk protein represents 10-30 wt% based on the total weight of the protein component.

2. Nutritional composition according to any preceding claim, wherein the protein component provides at least 15 EN% based on the total energy of the nutritional composition.

3. Nutritional composition according to any of the preceding claims having a protein to water ratio of at least 2.0/10 [g/g].

4. Nutritional composition according to any preceding claim, wherein the lipid component provides at most 50 EN% based on the total energy of the nutritional composition.

5. Nutritional composition according to any preceding claim, wherein the carbohydrate component provides at least 25 EN% based on the total energy of the nutritional composition.

6. Nutritional composition according to any preceding claim, wherein
a) the protein component provides 15-25 EN% based on the total energy of the nutritional composition
b) the lipid component provides 40-50 EN% based on the total energy of the nutritional composition and
c) the carbohydrate component provides 30-40 EN% based on the total energy of the nutritional composition.

7. Nutritional composition according to any preceding claim being an o/w emulsion.

8. Nutritional composition according to any of the preceding claims having a pH of 7-9.

9. Nutritional composition according to any preceding claim further **characterized in** being nutritionally complete.

10. Process for making a nutritional composition comprising a protein component comprising collagen hydrolysate with an average molecular weight M_{w} of from 1000 Da to 6000 Da, a lipid component, a carbohydrate component, minerals, vitamins and water, wherein the protein component comprises at least two different protein sources, wherein the nutritional composition has a caloric density of at least 2 kcal/mL, wherein the nutritional composition comprises at least 14g protein per 100mL product, wherein the second protein source is milk proteins, wherein the collagen hydrolysate represents 70-90 wt% based on the total weight of the protein component, and wherein the milk protein represents 10-30 wt% based on the total weight of the protein component, the process comprising the following steps:
i. a first step, wherein water is provided and heated to a temperature of 50-70°C;
ii. a second step, wherein the carbohydrate component - or a fraction thereof - and, optionally, a stabilizer, such as MCC, is added;
iii. a third step, wherein the protein component is added;
iv. a fourth step, wherein the lipid component is added, preferably at an elevated temperature of 80-90°C, preferably in combination with a (further) emulsifier and / or stabiliser, such as soy lecithin and / or diacetyl tartric acid of glycerides (e.g. DATEM), and / or (destilled) monoglycerides;
v. a fifth step, wherein vitamins, minerals and, optionally, the remaining fraction of the carbohydrate component are added, optionally, any other powder ingredient may be added in the fifth step, e.g. fibres, aroma, and other additives. At any time before, while or after the fifth step, the mixture is allowed to cool to 55-65°C;
vi. a sixth step, wherein the pH of the mixture is adjusted to 6-9, preferably 7-8, e.g. 7;
vii. a seventh step, wherein the mixture is homogenized, e.g. at 60-65°C at a pressure of 100/50 bar;
viii. an eighth step, wherein the mixture is sterilized. The eighth step may comprise (8.a) a pre-heating step, (8.b) a heating step and (8.c) an additional homogenization step.

11. The process of claim 10, wherein step 8 is divided into:
a) a step 8.a, wherein the mixture is pre-heated to 70-100°C, preferably 85-95°C, e.g. for 2-5 minutes;
b) a step 8.b, wherein the mixture is heated to 130-140°C, preferably to 139-141°C, e.g. for 6-15 seconds; and
c) a step 8c, wherein the mixture is homogenized at a pressure of 40-200bar, preferably step 8.c is carried out at less than 90°C.

## Patentansprüche

1. Nährstoffzusammensetzung, umfassend eine Proteinkomponente, eine Lipidkomponente, eine Kohlenhydratkomponente, Mineralien, Vitamine und Wasser, wobei die Proteinkomponente mindestens zwei verschiedene Proteinquellen umfasst; wobei die Nährstoffzusammensetzung einen pH-Wert im Bereich von 5,5 bis 9,0 hat, wobei die Nährstoffzusammensetzung eine Kaloriendichte von mindestens 2 kcal/ml aufweist, wobei die Nährstoffzusammensetzung mindestens 14 g Protein pro 100 ml Produkt umfasst, und wobei
a) die erste Proteinquelle hydrolysiertes Kollagen mit einem durchschnittlichen Molekulargewicht M_{w} von 1000 Da bis 6000 Da ist
b) die Lipidkomponente mindestens 30 EN-% der Gesamtenergie der Zusammensetzung bereitstellt
c) die erste Proteinquelle 70 bis 90 Gew.-%, bezogen auf das Gesamtgewicht der Proteinkomponente, darstellt und
d) die zweite Proteinquelle Milchprotein ist, und wobei das Milchprotein 10 bis 30 Gew.-%, bezogen auf das Gesamtgewicht der Proteinkomponente, darstellt.

2. Nährstoffzusammensetzung nach einem der vorstehenden Ansprüche, wobei die Proteinkomponente mindestens 15 EN-%, bezogen auf die Gesamtenergie der Nährstoffzusammensetzung, bereitstellt.

3. Nährstoffzusammensetzung nach einem der vorstehenden Ansprüche mit einem Protein-Wasser-Verhältnis von mindestens 2,0/10 [g/g].

4. Nährstoffzusammensetzung nach einem der vorstehenden Ansprüche, wobei die Lipidkomponente höchstens 50 EN-%, bezogen auf die Gesamtenergie der Nährstoffzusammensetzung, bereitstellt.

5. Nährstoffzusammensetzung nach einem der vorstehenden Ansprüche, wobei die Kohlenhydratkomponente mindestens 25 EN-%, bezogen auf die Gesamtenergie der Nährstoffzusammensetzung, bereitstellt.

6. Nährstoffzusammensetzung nach einem der vorstehenden Ansprüche, wobei
a) die Proteinkomponente 15 bis 25 EN-%, bezogen auf die Gesamtenergie der Nährstoffzusammensetzung, bereitstellt
b) die Lipidkomponente 40 bis 50 EN-%, bezogen auf die Gesamtenergie der Nährstoffzusammensetzung, bereitstellt, und
c) die Kohlenhydratkomponente 30 bis 40 EN-%, bezogen auf die Gesamtenergie der Nährstoffzusammensetzung, bereitstellt.

7. Nährstoffzusammensetzung nach einem der vorstehenden Ansprüche, die eine Öl/Wasser-Emulsion ist.

8. Nährstoffzusammensetzung nach einem der vorstehenden Ansprüche mit einem pH-Wert von 7 bis 9.

9. Nährstoffzusammensetzung nach einem der vorstehenden Ansprüche, ferner **dadurch gekennzeichnet, dass** sie diätetisch vollständig ist.

10. Verfahren zur Herstellung einer Nährstoffzusammensetzung, umfassend eine Proteinkomponente, umfassend Kollagenhydrolysat mit einem durchschnittlichen Molekulargewicht M_{w} von 1000 Da bis 6000 Da, eine Lipidkomponente, eine Kohlenhydratkomponente, Mineralien, Vitamine und Wasser, wobei die Proteinkomponente mindestens zwei verschiedene Proteinquellen umfasst, wobei die Nährstoffzusammensetzung eine Kaloriendichte von mindestens 2 kcal/ml aufweist, wobei die Nährstoffzusammensetzung mindestens 14 g Protein pro 100 ml Produkt umfasst, wobei die zweite Proteinquelle Milchprotein ist, wobei das Kollagenhydrolysat 70 bis 90 Gew.-%, bezogen auf das Gesamtgewicht der Proteinkomponente, darstellt und wobei das Milchprotein 10 bis 30 Gew.-%, bezogen auf das Gesamtgewicht der Proteinkomponente darstellt, wobei das Verfahren die folgenden Schritte umfasst:
i. einen ersten Schritt, wobei Wasser bereitgestellt und auf eine Temperatur von 50 bis 70 °C erwärmt wird;
ii. einen zweiten Schritt, wobei die Kohlenhydratkomponente - oder eine Fraktion davon - und wahlweise ein Stabilisator wie MCC zugegeben werden;
iii. einen dritten Schritt, wobei die Proteinkomponente zugegeben wird;
iv. einen vierten Schritt, wobei die Lipidkomponente zugegeben wird, vorzugsweise bei einer erhöhten Temperatur von 80 bis 90 °C, vorzugsweise in Kombination mit einem (weiteren) Emulgator und/oder Stabilisator wie Sojalecithin und/oder Diacetylweinsäure von Glyceriden (z. B. DATEM) und/oder (destillierten) Monoglyceriden;
v. einen fünften Schritt, wobei Vitamine, Mineralien und wahlweise die verbleibende Fraktion der Kohlenhydratkomponente zugegeben werden, wahlweise ein beliebiger anderer Pulverbestandteil im fünften Schritt zugegeben werden kann, z. B. Fasern, Aroma und andere Zusatzstoffe; zu jeder Zeit vor, während oder nach dem fünften Schritt, die Mischung auf 55 bis 65 °C abkühlen gelassen wird;
vi. einen sechsten Schritt, wobei der pH-Wert der Mischung auf 6 bis 9, vorzugsweise 7 bis 8, z. B. 7 eingestellt wird;
vii. einen siebten Schritt, wobei die Mischung z. B. bei 60 bis 65 °C bei einem Druck von 100/50 bar homogenisiert wird;
viii. einen achten Schritt, wobei die Mischung sterilisiert wird; der achte Schritt kann (8.a) einen Vorwärmungsschritt, (8.b) einen Erwärmungsschritt und (8.c) einen zusätzlichen Homogenisierungsschritt umfassen.

11. Verfahren nach Anspruch 10, wobei Schritt 8 aufgeteilt ist in:
a) einen Schritt 8.a, wobei die Mischung auf 70 bis 100 °C, vorzugsweise 85 bis 95 °C, z. B. für 2 bis 5 Minuten vorgewärmt wird;
b) einen Schritt 8.b, wobei die Mischung auf 130 bis 140 °C, vorzugsweise 139 bis 141 °C, z. B. für 6 bis 15 Minuten erwärmt wird; und
c) einen Schritt 8c, wobei die Mischung bei einem Druck von 40 bis 200 bar homogenisiert wird, vorzugsweise Schritt 8.c bei weniger als 90 °C durchgeführt wird.

## Revendications

1. Composition nutritionnelle comprenant un composant protéique, un composant lipidique, un composant glucidique, des minéraux, des vitamines et de l'eau, dans laquelle le composant protéique comprend au moins deux sources de protéines différentes ; dans laquelle la composition nutritionnelle a un pH dans la plage de 5,5 à 9,0, dans laquelle la composition nutritionnelle a une densité calorique d'au moins 2 kcal/mL, dans laquelle la composition nutritionnelle comprend au moins 14 g de protéines pour 100 mL de produit, et dans laquelle
a) la première source de protéines est du collagène hydrolysé avec un poids moléculaire moyen M_{w} de 1000 Da à 6000 Da
b) le composant lipidique fournit au moins 30 % en énergie de l'énergie totale de la composition
c) la première source de protéines représente 70 à 90 % en poids sur la base du poids total du composant protéique et
d) la deuxième source de protéines est la protéine du lait et dans laquelle la protéine du lait représente 10 à 30 % en poids sur la base du poids total du composant protéique.

2. Composition nutritionnelle selon l'une quelconque des revendications précédentes, dans laquelle le composant protéique fournit au moins 15 % en énergie sur la base de l'énergie totale de la composition nutritionnelle.

3. Composition nutritionnelle selon l'une quelconque des revendications précédentes ayant un rapport protéine/eau d'au moins 2,0/10 [g/g].

4. Composition nutritionnelle selon l'une quelconque des revendications précédentes, dans laquelle le composant lipidique fournit au plus 50 % en énergie sur la base de l'énergie totale de la composition nutritionnelle.

5. Composition nutritionnelle selon l'une quelconque des revendications précédentes, dans laquelle le composant glucidique fournit au moins 25 % en énergie sur la base de l'énergie totale de la composition nutritionnelle.

6. Composition nutritionnelle selon l'une quelconque des revendications précédentes, dans laquelle
a) le composant protéique fournit 15 à 25 % en énergie sur la base de l'énergie totale de la composition nutritionnelle
b) le composant lipidique fournit 40 à 50 % en énergie sur la base de l'énergie totale de la composition nutritionnelle et
c) le composant glucidique fournit 30 à 40 % en énergie sur la base de l'énergie totale de la composition nutritionnelle.

7. Composition nutritionnelle selon l'une quelconque des revendications précédentes étant une émulsion H/E.

8. Composition nutritionnelle selon l'une quelconque des revendications précédentes ayant un pH de 7 à 9.

9. Composition nutritionnelle selon l'une quelconque des revendications précédentes **caractérisée en outre en ce qu'**elle est nutritionnellement complète.

10. Procédé de préparation d'une composition nutritionnelle comprenant un composant protéique comprenant un hydrolysat de collagène avec un poids moléculaire moyen M_{w} de 1000 Da à 6000 Da, un composant lipidique, un composant glucidique, des minéraux, des vitamines et de l'eau, dans lequel le composant protéique comprend au moins deux sources de protéines différentes, dans lequel la composition nutritionnelle a une densité calorique d'au moins 2 kcal/mL, dans lequel la composition nutritionnelle comprend au moins 14 g de protéines pour 100 mL de produit, dans lequel la deuxième source de protéines est des protéines de lait, dans lequel l'hydrolysat de collagène représente 70 à 90 % en poids sur la base du poids total du composant protéique, et dans lequel la protéine de lait représente 10 à 30 % en poids sur la base du poids total du composant protéique, le procédé comprenant les étapes suivantes :
i. une première étape, dans laquelle de l'eau est fournie et chauffée à une température de 50 à 70 °C ;
ii. une deuxième étape, dans laquelle le composant glucidique - ou une fraction de celui-ci - et, optionnellement, un stabilisant, tel que MCC, est ajouté ;
iii. une troisième étape, dans laquelle le composant protéique est ajouté ;
iv. une quatrième étape, dans laquelle le composant lipidique est ajouté, de préférence à une température élevée de 80 à 90 °C, de préférence en combinaison avec un (autre) émulsifiant et/ou stabilisant, tel que la lécithine de soja et/ou l'acide diacétyltartrique des glycérides (par ex. DATEM) et/ou des monoglycérides (distillés) ;
v. une cinquième étape, dans laquelle les vitamines, les minéraux et, optionnellement, la fraction restante du composant glucidique sont ajoutés, optionnellement, tout autre ingrédient en poudre peut être ajouté dans la cinquième étape, par exemple des fibres, de l'arôme, et autres additifs ; à tout moment avant, pendant ou après la cinquième étape, on laisse le mélange refroidir à 55-65 °C ;
vi. une sixième étape, dans laquelle le pH du mélange est ajusté à 6-9, de préférence à 7-8, par exemple, 7 ;
vii. une septième étape, dans laquelle le mélange est homogénéisé, par exemple, à 60-65 °C à une pression de 100/50 bars ;
viii. une huitième étape, dans laquelle le mélange est stérilisé ; la huitième étape peut comprendre (8.a) une étape de préchauffage, (8.b) une étape de chauffage et (8.c) une étape d'homogénéisation supplémentaire.

11. Procédé selon la revendication 10, dans lequel l'étape 8 est divisée en :
a) une étape 8.a, dans laquelle le mélange est préchauffé à 70-100 °C, de préférence à 85-95 °C, par exemple, pendant 2 à 5 minutes ;
b) une étape 8.b, dans laquelle le mélange est chauffé à 130-140 °C, de préférence à 139-141 °C, par exemple, pendant 6 à 15 secondes ; et
c) une étape 8c, dans laquelle le mélange est homogénéisé à une pression de 40 à 200 bars, de préférence, l'étape 8.c est effectuée à moins de 90 °C.
